# EUROPEAN PATENT SPECIFICATION

(11) **EP 1 005 875 B1**
(45) Date of publication and mention of the grant of the patent: **14.09.2005**
(21) Application number: 99309480.4
(22) Date of filing: 26.11.1999
(51) Int. Cl.: A61M 5/145

(54) **Liquid infusion apparatus**
Liquidinfusionsvorrichtung
Dispositif de perfusion de liquide

(30) Priority: 01.12.1998 JP 35545698
(43) Date of publication of application: 07.06.2000
(73) Proprietor: JAPAN SERVO CO. LTD., Tokyo 101 (JP); JMS Co., Ltd., Hiroshima-shi, Hiroshima 730-0812 (JP)
(72) Inventor: Hayashi, Shigeo, c/o JMS Co., Ltd., Yamagata-gun, Hiroshima (JP); Kojima, Seiji, c/o Japan Servo Co., Ltd., Kiryu-shi, Gunma (JP)
(74) Representative: Loven, Keith James

(56) References cited:
- DATABASE WPI Section Ch, Week 199834 Derwent Publications Ltd., London, GB; Class B07, AN 1998-398820 XP002137642 -& WO 98 30260 A (JAPAN SERVO CO LTD), 16 July 1998 (1998-07-16)

## Description

The present invention relates to a liquid infusion apparatus, and more particularly, relates to a liquid infusion apparatus for infusing liquid such as a liquid medicine or blood into a human body by pushing a plunger into a syringe by the rotation of a prim mover, said liquid infusion apparatus having a holding device for holding said plunger.

The present invention is based on the invention of the Japanese Patent Laid-Open No. 192399/1998.

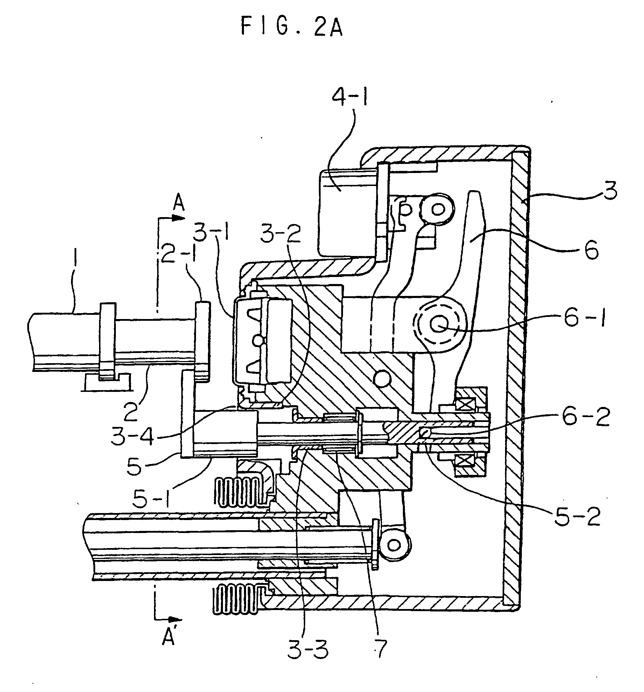

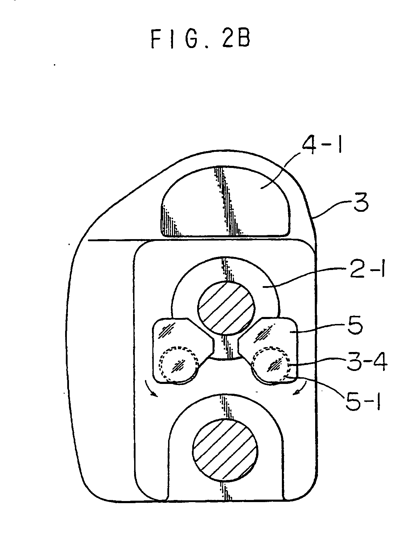

FIGS. 2A and 2B show a liquid infusion apparatus of said prior art, wherein reference numeral 1 is a syringe, 2 is a plunger, 2-1 is a flange of the plunger 2, 3 is a slider, 3-1 is a contact face of the slider 3, 3-2 is a front wall of the slider 3, 4-1 is a push button, 5 is a holding arm for holding said flange 2-1 of the plunger 2, which is movable in a moving direction of said slider 3 and rotatable around the axis thereof, 5-1 is a supporting shaft of the holding arm 5, 5-2 is a screw grove formed on the supporting shaft 5-1, 3-3 is a bearing formed on the slider 3 for supporting said supporting shaft 5-1, 3-4 is a through hole formed on said front wall 3-2 for the supporting shaft 5-1, 6 is a lever for driving said supporting shaft 5-1, 6-1 is a pivot shaft for the lever 6, 6-2 is an engaging pin projected from the side surface of the lever 6 for engaging with the screw groove 5-2, and 7 is a return spring for the supporting shaft 5-1.

Two of said holding arms 5 are provided so as to be rotated in the opposite directions, respectively at the same time by the push button 4-1 to open the holding arms 5 or to close the same: The flange 2-1 of the plunger 2 is urged toward the contact face 3-1 of the slider 3 when the holding arms 5 is closed as shown in FIG. 2B and moved toward the slider 3 by the return spring 7.

FIGS. 2A and 2B show a state that the flange 2-1 of the plunger 2 is held by the holding arms 5, and FIGS. 3A and 3B show a state that the flange 2-1 of the plunger 2 is not yet held by the holding arms 5.

FIG. 4 is a state that the flange 2-1 of the plunger 2 is held and brought into contact with the contact face 3-1 of the slider 3 by the action of the holding arms 5.

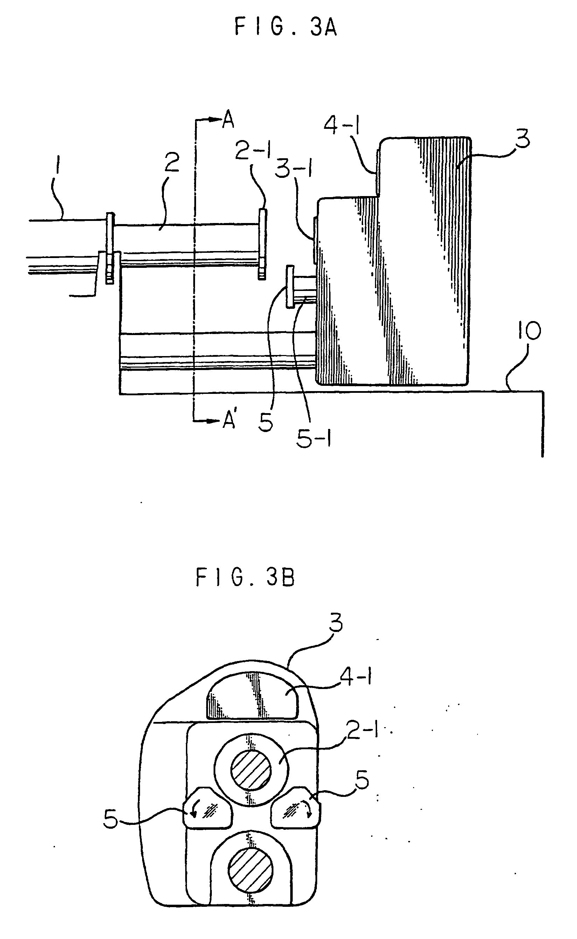

In the state shown in FIGS. 3A and 3B, the holding arms 5 are not brought into contact with the flange 2-1 of the plunger 2 and when the push button 4-1 is pushed, the lever 6 linked to the push button 4-1 is rotated centering around the pivot shaft 6-1, so that the supporting shaft 5-1 of the holding arms 5 is moved leftwards against the spring force of the return spring 7, and at the same time, the supporting shaft 5-1 of the holding arms 5 for holding the flange 2-1 of the plunger 2 is rotated and the holding arms 5 are opened as shown in FIG. 3B by the action of the screw groove 5-2 and the engaging pin 6-2 engaged thereto.

Thereafter, the flange 2-1 of the plunger is positioned between the holding arms 5 and the contact face 3-1 of the slider 3.

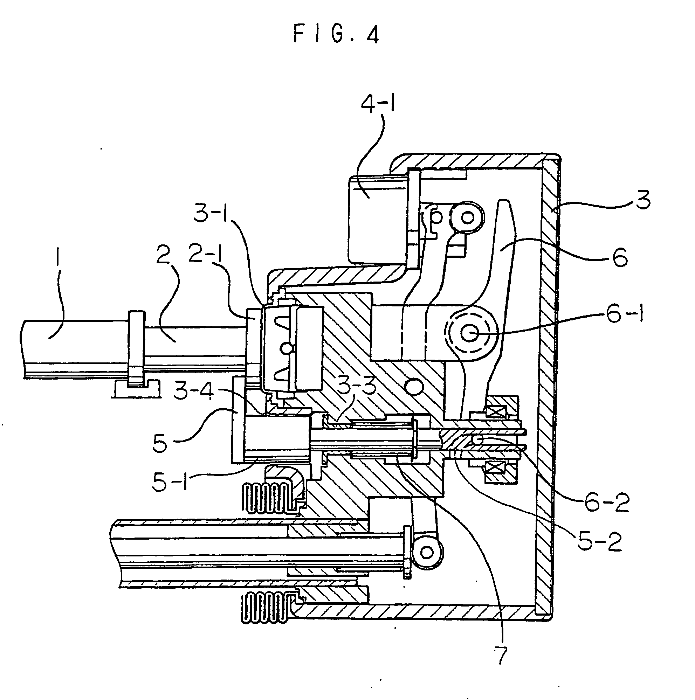

When the push button 4-1 is retracted, as shown in FIG. 2A, the lever 6 is returned and the supporting shaft 5-1 of the holding arms 5 is moved in the rightward direction by the spring force of the return spring 7, so that the supporting arms 5 are rotated and closed as shown in FIG. 2B by the action of the screw groove 5-2 and the engaging pin 6-2 engaged thereto. Accordingly, it is possible to hold the flange 2-1 of the plunger 2 and to bring the flange 2-1 of the plunger 2 into contact with the contact face 3-1 of the slider 3 positively irrespective of the thickness of the flange 2-1 of the plunger 2, as shown in FIG. 4.

It is understood that the holding arms 5 are rotated in the reverse direction with each other, but operated similar to each other.

As stated above, according to the conventional liquid infusion apparatus, the workability can be enhanced when the syringe 1 is installed. However, in case that a negative pressure is applied to the syringe 1, the plunger 2 is sucked into the syringe 1 if the sucking force is larger than the spring force of the return spring 7, so that undesirable exceed liquid is injected into the human body.

An object of the present invention is to obviate such defect.

The scope of the liquid infusion apparatus of the present invention is defined by the appended claims.

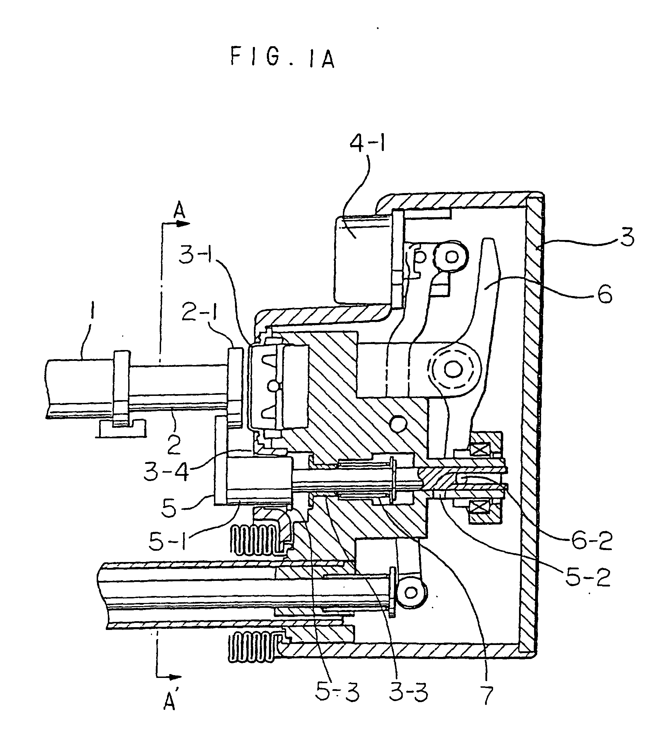

FIG. 1A illustrates a vertical sectional side view of an essential portion of a liquid infusion apparatus according to an embodiment of the present invention;

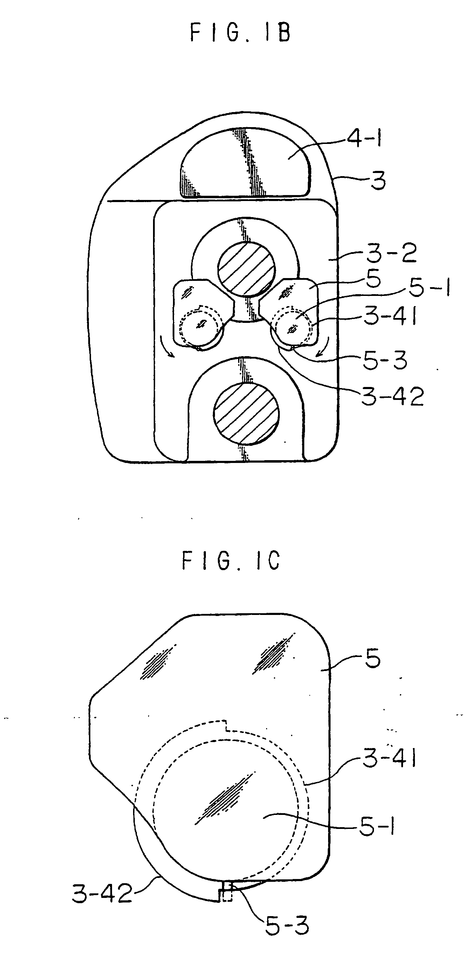

FIG. 1B illustrates a front view, taken along lines A-A' of FIG. 1A;
FIG. 1C illustrates an enlarged view of a portion of FIG. 1B;
FIG. 2A illustrates a vertical sectional side view of the construction of a conventional liquid infusion apparatus;
FIG. 2B illustrates a front view, taken along lines A-A' of FIG. 2A;
FIG. 3A illustrates a side view of an essential portion of the conventional liquid infusion apparatus in a state that a flange of a plunger is not yet held by a slider;
FIG. 3B illustrates a front view, taken along lines A-A' of FIG. 3A; and
FIG. 4 illustrates a vertical sectional side view of an essential portion of the conventional liquid infusion apparatus in a state that the flange of the plunger is held by the slider.

Now, an embodiment of this invention will be described by referring to drawings.

According to the present invention, as shown in FIGS. 1A to 1C, the through hole 3-4 is formed of a semi-circular hole portion 3-41 of a small diameter and a semi-circular hole portion 3-42 of a large diameter, the supporting shaft 5-1 of said holding arm 5 being able to pass through said through hole 3-4.

A stopper pin 5-3 is provided on the outer peripheral surface portion of said supporting shaft 5-1 so as to extend radially outwardly, the length of said stopper pin being such a value that it can pass through said semi-circular hole portion 3-42 of the large diameter, but cannot pass through said semi-circular hole portion 3-41 of the small diameter. The stopper pin 5-3 is so positioned that it is brought into contact with the inside of the front wall of the slider 3 at which the semi-circular hole portion 3-41 of the small diameter is formed when the flange 2-1 of the plunger 2 held by the holding arms 5 is positioned near the contact face 3-1 of the slider 3. The stopper pin 5-3 is positioned at the hole portion 3-42 of the large diameter by the rotation of the supporting shaft 5-1 of the holding arm by the arrangement of the screw groove 5-2 provided on the supporting shaft 5-1 of the holding arms 5 and the engaging pin 6-2 of the lever 6 when the holding arms 5 is moved apart from the contact face 3-1 of the slider 3 by the lever 6.

That is, according to the present invention, when the push button 4-1 is pushed, the supporting shaft 5-1 of the holding arms 5 is moved axially passing through the through hole 3-4 while rotating by the action of the screw groove 5-2 and the engaging pin 6-2 of the lever 6. On the contrary, in case that the negative pressure is applied to the plunger 2, the plunger 2 cannot be sucked into the syringe 1, because the holding arms 5 are not rotated and the stopper pin 5-3 is contacted with the inside of the front wall of the slider 3 at which the semi-circular hole portion 3-41 of the small diameter is formed. This means that the stopper pin 5-3 cannot be passed through the semi-circular hole portion 3-42 of the large diameter and that the supporting shaft 5-1 of the holding arms 5 cannot be moved leftwards linearly in FIG. 1A.

As stated above, in the liquid infusion apparatus according to the present invention, it is possible to prevent the undesirable exceed liquid from being injected into the human body, even if the negative pressure is applied to the plunger 2.

## Claims

1. A liquid infusion apparatus having a slider (3) adapted to be moved linearly by a prime mover so as to push a plunger (2) into a syringe (1) and exhaust liquid from the syringe (1), and holding means (5) movable relative to the slider (3) in the direction of movement of the plunger (2) for holding the plunger (2) near the slider (3), the holding means (5) also being movable so as to be disengaged from the plunger (2) when the holding means (5) is moved so as to be separated from the slider (3) and to be engaged with the plunger (2) when the holding means (5) is moved so as to approach the slider (3), **characterised by** a blocking device (5-3, 3-41, 3-42) for preventing the holding means (5) from being disengaged from the plunger (2) when the plunger (2) is moved from a position near the slider (3) to a position remote from the slider (3), whereby to prevent negative pressure on a syringe disposed in the liquid infusion apparatus from drawing the plunger (2) into the syringe and unintentionally expelling liquid from the syringe.

2. The liquid infusion apparatus as claimed in Claim 1, wherein said holding means (5) holds the plunger (2) when the plunger is near said slider (3), but does not hold the plunger (2) when the plunger is separated from the slider (3).

3. The liquid infusion apparatus as claimed in Claim 2, wherein said holding means (5) is rotatable so as to be disengaged from the plunger (2) when the holding means (5) is separated from the slider (3), and to be engaged with the plunger (2) when the holding means (5) is near the slider (3).

4. The liquid infusion apparatus as claimed in Claim 1 or 2 wherein the plunger (2) has a flange (2-1), and the holding means (5) is adapted to hold the flange (2-1) of the plunger (2) when engaged with the plunger.

5. The liquid infusion apparatus as claimed in Claim 1, wherein the plunger (2) has a flange (2-1), and the holding means (5) is adapted to hold the flange (2-1) of the plunger (2) when engaged with the plunger, said holding means (5) adapted to hold the flange (2-1) when said plunger is near said slider (3), but not to hold said flange (2-1) of the plunger (2) when said plunger is separated from the slider (3).

6. The liquid infusion apparatus as claimed in Claim 1, wherein the plunger (2) has a flange (2-1), and the holding means (5) is adapted to hold the flange (2-1) of the plunger (2) when the plunger is near said slider (3), but not to hold the flange (2-1) of the plunger (2) when the plunger is separated from the slider (3), said holding means (5) being rotatable so as to be disengaged from the flange (2-1) of the plunger (2) when the holding means (5) is moved so as to be separated from the slider, and to be engaged with the flange (2-1) of the plunger (2) when the holding means (5) is moved so as to be near the slider (3).

## Patentansprüche

1. Flüssigkeitsinfusionsvorrichtung mit einem Schieber (3), der durch einen Hauptbewegungsantrieb linear bewegbar ist, um einen Kolben (2) in eine Spritze (1) zu drücken und eine Flüssigkeit aus der Spritze (1) herauszufördern, und einer Halteeinrichtung (5), die relativ zu dem Schieber (3) in die Richtung der Bewegung des Kolbens (2) bewegbar ist, um den Kolben (2) nahe des Schiebers (3) zu halten, wobei die Halteeinrichtung (5) ferner derart bewegbar ist, um sich außer Eingriff mit dem Kolben (2) zu befinden, wenn die Halteeinrichtung (5) bewegt wird, um von dem Schieber (3) getrennt zu werden, und sich in Eingriff mit dem Kolben (2) befindet, wenn die Halteeinrichtung (5) bewegt wird, um sich dem Schieber (3) zu nähern, **gekennzeichnet durch** eine Blockiereinrichtung (5-3, 3-41, 3-42) zum Verhindern, daß die Halteeinrichtung (5) außer Eingriff mit dem Kolben (2) gelangt, wenn der Kolben (2) von einer Position nahe des Schiebers (3) in eine Position entfernt von dem Schieber (3) bewegt wird, wodurch verhindert wird, daß ein negativer Druck an der in der Flüssigkeitsinfusionsvorrichtung angeordneten Spritze den Kolben (2) in die Spritze zieht und unbeabsichtigt Flüssigkeit aus der Spritze abgibt.

2. Flüssigkeitsinfusionsvorrichtung nach Anspruch 1, bei der die Halteeinrichtung (5) den Kolben (2) hält, wenn sich der Kolben nahe des Schiebers (3) befindet, jedoch den Kolben (2) nicht hält, wenn der Kolben von dem Schieber (3) getrennt ist.

3. Flüssigkeitsinfusionsvorrichtung nach Anspruch 2, bei der die Halteeinrichtung (5) derart drehbar ist, daß sich diese außer Eingriff mit dem Kolben (2) befindet, wenn die Halteeinrichtung (5) von dem Schieber (3) getrennt ist, und sich mit dem Kolben (2) in Eingriff befindet, wenn die Halteeinrichtung (5) sich nahe des Kolbens (3) befindet.

4. Flüssigkeitsinfusionsvorrichtung nach Anspruch 1 oder 2, bei der der Kolben (2) einen Flansch (2-1) aufweist und die Halteeinrichtung (5) derart ausgebildet ist, um den Flansch (2-1) des Kolbens (2) zu halten, wenn sich diese in Eingriff mit dem Kolben befindet.

5. Flüssigkeitsinfusionsvorrichtung nach Anspruch 1, bei der der Kolben (2) einen Flansch (2-1) aufweist und die Halteeinrichtung (5) derart ausgebildet ist, um den Flansch (2-1) des Kolbens (2) zu halten, wenn sich diese in Eingriff mit dem Kolben befindet, wobei die Halteeinrichtung (5) ferner derart ausgebildet ist, um den Flansch (2-1) zu halten, wenn sich der Kolben nahe des Schiebers (3) befindet, jedoch den Flansch (2-1) des Kolbens (2) nicht zu halten, wenn der Kolben von dem Schieber (3) getrennt ist.

6. Flüssigkeitsinfusionsvorrichtung nach Anspruch 1, bei der der Kolben (2) einen Flansch (2-1) aufweist und die Halteeinrichtung (5) derart ausgebildet ist, um den Flansch (2-1) des Kolbens (2) zu halten, wenn sich der Kolben nahe des Schiebers (3) befindet, jedoch den Flansch (2-1) des Kolbens (2) nicht zu halten, wenn der Kolben von dem Schieber (3) getrennt ist, wobei die Halteeinrichtung (5) derart drehbar ist, daß sich diese außer Eingriff mit dem Flansch (2-1) des Kolbens (2) befindet, wenn die Halteeinrichtung (5) bewegt wird, um von dem Schieber (3) getrennt zu werden, und sich in Eingriff mit dem Flansch (2-1) des Kolbens (2) befindet, wenn die Halteeinrichtung (5) bewegt wird, um sich nahe dem Schieber (3) zu befinden.

## Revendications

1. Appareil d'infusion de liquide ayant une coulisse (3) adaptée pour être déplacée linéairement par un moteur d'entraînement de façon à pousser un plongeur (2) dans une seringue (1) et à décharger du liquide à partir de la seringue (1), et un moyen de support (5) déplaçable par rapport à la coulisse (3) dans la direction de mouvement du plongeur (2) pour maintenir le plongeur (2) près de la coulisse (3), le moyen de support (5) étant également déplaçable de façon à être désengagé du plongeur (2) lorsque le moyen de support (5) est déplacé de façon à être séparé de la coulisse (3) et à être engagé avec le plongeur (2) lorsque le moyen de support (5) est déplacé de façon à s'approcher de la coulisse (3), **caractérisé par** un dispositif de blocage (5-3, 3-41, 3-42) pour empêcher le moyen de support (5) d'être désengagé du plongeur (2) lorsque le plongeur (2) est déplacé d'une position proche de la coulisse (3) à une position éloignée de la coulisse (3), pour empêcher de cette façon une pression négative sur une seringue disposée dans l'appareil d'infusion de liquide de tirer le plongeur (2) dans la seringue et d'expulser de façon non intentionnelle du liquide à partir de la seringue.

2. Appareil d'infusion de liquide selon la revendication 1, dans lequel ledit moyen de support (5) maintient le plongeur (2) lorsque le plongeur est près de ladite coulisse (3), mais ne maintient pas le plongeur (2) lorsque le plongeur est séparé de la coulisse (3).

3. Appareil d'infusion de liquide selon la revendication 2, dans lequel ledit moyen de support (5) est apte à tourner de façon à être désengagé du plongeur (2) lorsque le moyen de support (5) est séparé de la coulisse (3), et à être engagé avec le plongeur (2) lorsque le moyen de support (5) est près de la coulisse (3).

4. Appareil d'infusion de liquide selon l'une des revendications 1 ou 2, dans lequel le plongeur (2) a une bride (2-1), et le moyen de support (5) est adapté pour maintenir la bride (2-1) du plongeur (2) lorsqu'elle est engagée avec le plongeur.

5. Appareil d'infusion de liquide selon la revendication 1, dans lequel le plongeur (2) a une bride (2-1), et le moyen de support (5) est adapté pour maintenir la bride (2-1) du plongeur (2) lorsqu'elle est engagée avec le plongeur, ledit moyen de support (5) étant adapté pour maintenir la bride (2-1) lorsque ledit plongeur est près de ladite coulisse (3), mais non pour maintenir ladite bride (2-1) du plongeur (2) lorsque ledit plongeur est séparé de la coulisse (3).

6. Appareil d'infusion de liquide selon la revendication 1, dans lequel le plongeur (2) a une bride (2-1), et le moyen de support (5) est adapté pour maintenir la bride (2-1) du plongeur (2) lorsque le plongeur est près de ladite coulisse (3), mais non pour maintenir ladite bride (2-1) du plongeur (2) lorsque le plongeur est séparé de la coulisse (3), ledit moyen de support (5) étant apte à tourner de façon à être désengagé de la bride (2-1) du plongeur (2) lorsque le moyen de support (5) est déplacé de façon à être séparée de la coulisse, et à être engagé avec la bride (2-1) du plongeur (2) lorsque le moyen de support (5) est déplacé de façon à être près de la coulisse (3).
